# EUROPEAN PATENT SPECIFICATION

(11) **EP 1 234 885 B1**
(45) Date of publication and mention of the grant of the patent: **23.07.2008**
(21) Application number: 01103844.5
(22) Date of filing: 16.02.2001
(51) Int. Cl.: C12P 17/06, C12P 7/02, C12P 7/26, C07C 69/013, C07F 7/18, C12P 9/00

(54) **A process for the preparation of beta-hydroxy-delta-lactone using novel intermediates**
Verfahren zur Herstellung eines Beta-Hydroxy-Delta-Lactons mittels neuer Zwischenprodukte
Procédé de préparation de béta-hydroxy-delta-lactone en utilisant des produits intermédiaires nouveaux

(43) Date of publication of application: 28.08.2002
(73) Proprietor: Council of Scientific & Industrial Research, New Delhi 110001 (IN)
(72) Inventor: Sandeep, Raghunath Ghorpade, 008, Maharashtra (IN); Uttam, Ramrao Kalkote, 008, Maharashtra (IN); Subhash, Prataprao Chavan, 008, Maharashtra (IN); Sunil, Ramchandra Bhide, 008, Maharashtra (IN); Thottappillil, Ravindranathan, 008, Maharashtra (IN)
(74) Representative: Henkel, Feiler & Hänzel

(56) References cited:
- WO-A-93/06235
- WIRZ B ET AL: "Multiselective enzymatic reactions for the synthesis of protected homochiral cis- and trans-1,3,5-cyclohexanetriols" TETRAHEDRON: ASYMMETRY,ELSEVIER SCIENCE PUBLISHERS, AMSTERDAM,NL, vol. 11, no. 20, 20 October 2000 (2000-10-20), pages 4171-4178, XP004221070 ISSN: 0957-4166
- SUEMUNE H ET AL: "ASYMMETRIC HYDROLYSIS OF CIS,CIS-5-BENZYLOXY-1,3-DIACETOXY-CYCLOHEX ANE AND ITS APPLICATION TO THE SYNTHESIS OF CHIRAL LACTONE MOIETY IN COMPACTIN" TETRAHEDRON: ASYMMETRY,ELSEVIER SCIENCE PUBLISHERS, AMSTERDAM,NL, vol. 1, no. 7, 1990, pages 425-428, XP000986250 ISSN: 0957-4166

## Description

This invention relates to a **process for the preparation of optically active 6-hydroxymethyl-4-(*tert*-butyldimethylsilyloxy)-(4R,6S)-tetrahydro-2*H*-2-pyranone** (β-hydroxy-δ-lactone) having formula **1.**

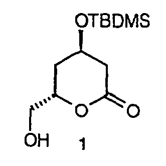

More particularly it relates to a process for the preparation of the said compound using *Cis,cis*-3,5-di(methylcarbonyloxy)cyclohexylacetate having formula **2**

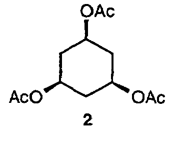

The compound β-hydroxy-δ-lactone (**1**) is an important intermediate in the synthesis of biologically active drugs e.g. compactin, atorvastatin, fluvastatin , cholesterol lowering drugs.

Hitherto known processes for the synthesis of β-hydroxy-δ-lactone (**1**) involves
a) Addition of lithium enolate of ethylacetate to (S)-2,2-dimethyl-1,3-dioxlane-4-ethanol, which in derived from L-malic acid, followed by acid treatment. (T. Rosen, M.J. Taschner & C.H. Heathcock, J. Org. Chem., 1984, 49, 3994-4003)
b) Multistep chemical manipulation of tri-acetyl-D-glucal (T. Rosen, M.J. Taschner, C.H. Heathcock, J. Org. Chem., 1984, 49, 3994; F.G. Kathawala, Mountain Lake N.J. USP 4739,073)
c) Coupling of (S)-2,2-dimethyl-1,3-dioxalane-4-ethanal with optically active (R)-methyl-p-tolylsulphoxide which in turn obtained by oxidation of methyl-p-tolylsulphide with baker yeast, followed by desulphurization and few chemical manipulation (J. Beecher, I. Brackerridge, S.M. Roberts, J. Tang & A.J. Willetts, J. Chem. Soc. Perkin Tran.I 1995, 1641; Tetrahedron 1995, 51, 13217)
d) Deprotection and hydrolysis of 6-cyanomethyl-2,2-dimethyl-1,3-dioxane-4-acetate, which in turn obtained by two carbon homologation on optically active ethyl-3-hydroxy-4-cyanobutyrate and followed by stereoselective reduction (P.L. Brower, D.E. Butler, C.F. Deering, T.V. Le, A. Millar, T.N. Nanninga & B.D. Roth, Tet. Lett, 1992, 33, 2279-82
e) Racemic and optically active β-hydroxy-δ-lactone from *cis*-cyclohexane-1,3,5-triol (K. Prasad & O. Repic, Tet. Lett., 1984, 25, 2435-38; H. Suemune, M. Takahashi, S. Maeda, Z. Fxi & K. Sakai, Tet. Asymm. 1990, 1, 425-8, M. Canda, V.Eyken & M. Vandewalle, Tet. Asymmetry 1990, 1, 17-20).
f) Enzymatic kinetic resolution of racemic β-hydroxy-δ-lactone by transesterification with vinyl acetate in THF using *Chromobacteriun viscosum* lipase as catalyst at 40 °C. [Crosby, J. B.; Andrew, J. H.; John, A. L. WO 9306235 A1 CA 119:93692 (1993)]
g) Chemoenzymatic route involving kinetic resolution through lactone formation in ether catalyzed by PPL [Bonini, C.; Pucci, P.; Viggiani, L. J. Org. Chem. 1991, 56, 4050]
h) Chemoenzymatic route involing enzymatic desymmetrization of intermediate diacetate, followed by chemical conversions. [Bonni, C.; Racioppi, R.; Righi, G.; Viggiani, L. J. Org. Chem. 1991, 58, 802]
i) Chemoenzymatic synthesis starting from endohydroxylacto which is obtained by enzymatic resolution [MaCague, R.; Olivo, H. F.; Roberts, S. M. Tetrahedron Lett. 1993, 34, 3785]
j) Diastereoselective synthesis of lactone based on Eu(fod)₃ catalyzed highly diastereoselective [4+2] cycloaddition of 1-methoxybuta-1,3-diene to (2*R*)-N-glyoxyloxyborane-10,2-sultam and further chemical transformations [ Bauer, T.; Kozak, J.; Chauis, C.; Jurczak, J. J. Chem. Soc.; Chem. Commun. 1990, 1178 and Tetrahedron: Asymmetry 1996, 7, 1391]
k) Chiral synthesi using (R)-O-benzylglycidol as starting material [Takano, S.; Shimazaki, Y.; Sekiguchi, Y.; Ogasawara, K. Synthesis 1989, 539]
l) Asymmetric synthesis based on Red-Al promoted intramolecular reductive cleavage of Benzyl 4-hydroxy-2-butenyl ether structures. [Hatakeyama, S.; Satoh, K.; Takano, S. Tetrahedron Lett. 1993, 34, 7425]

The prior art processes have following drawbacks:
1. The processes use chemicals such as butyl lithium, lithium aluminum hydride, methoxy-diethylborane which are costly and difficult to handle and therefore make the process difficult.
2. All known process are however involves large number of synthetic steps resulting in low over all yields.

The main object of the present invention is to provide a new process for the preparation of β-hydroxy-δ-lactone (**1**), which obviates the drawbacks of the prior art processes and use cheaper and easily accessible chemicals.

Another object of the present invention is to provide (i) selective Baeyer-Villiger rearrangement of 3-hydroxy-5-t-butyldimethylsilyloxy-1-cyclohexanone (**9**) with chemical reagent or Baeyer Villiger oxidase and (ii) enantioselective hydrolysis of *cis*-3-(methylcarbonyloxy)-5-(tert-butyldimethylsilyloxy)cyclohexylacetate with enzyme.

### DETAILED DESCRIPTION OF THE INVENTION

Accordingly the present invention provides a process for the preparation of β-hydroxy-δ-lactone of formula **1** using novel intermediates which comprises
a) reacting a compound of formula **2** with a lipase enzyme in a buffer having 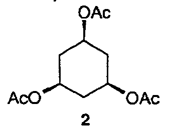 pH ranging between 5 to 7, at a temperature ranging from 25 to 30 °C for a period ranging between 19 to 30 hrs. extracting the reaction mixture with an organic solvent, removing the solvent by evaporation to obtain *cis,cis*-3-hydroxy-5-methylcarbonyloxy-cyclohexylacetate having formula (**3**), 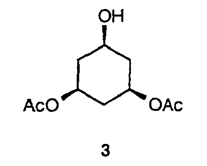
b) reacting a compound of formula **3** with *tert*-butyldimethylsilylchloride in an organic solvent in the presence of an organic base at a temperature ranging from -15 to 20°C for a period ranging from 6 to 12 hrs, separating the organic solvent, to obtain *cis,cis*-3-(methylcarbonyloxy)-5-(*tert*.butyldimethylsilyloxy)cyclohexylacetate having formula **4,** 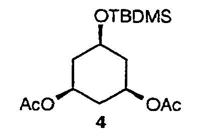
c) reacting a compound **4** with a lipase enzyme in a buffer having pH in the range of 5 to 8, at a temperature ranging from 25 to 30 °C for a period ranging between 24 to 60 hrs., extracting the mixture with an organic solvent, removing the solvent by evaporation and on column chromatography to obtain 3-hydroxy-5-(*tert*.butyldimethylsilyloxy)-(1*S*,3*R*,5*R*)-cyclohexylace- tate having formula **5,** 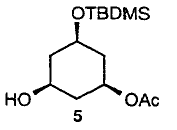
d) reacting a compound of formula **5** with dihydropyran in an organic solvent in the presence of p-toluene sulphonic acid at a temperature ranging from 5 to 10 °C for a period ranging from 2 to 5 hrs, quenching the above reaction with an aqueous sodium bicarbonate, separating the organic layer, drying, on evaporating and column chromatography to obtain 3-tetrahydro-2*H*-2-pyranyloxy-5-(*tert*.butyldimethylsilyloxy)-(1*S*,3*R*,5*R*)-cyclohexylacetate having formula **6,** 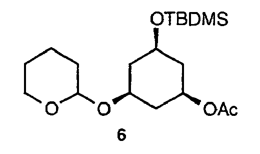
e) reacting a compound of formula **6** with an anhydrous potassium carbonate in methanol at room temperature for a period ranging from 2 to 6 hrs, evaporating the solvent, extracting with an organic solvent, washing with brine solution, drying, evaporating and column chromatography to obtain 3-tetrahydro-2*H*-2-pyranyloxy-5-(*tert*.butyldimethylsilyloxy)-(1*S*,3*R*,5*R*)-cyclohexan-1-ol having formula **7,** 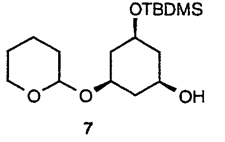
f) reacting a compound of formula **7** with pyridinium chlorochromate in an organic solvent at room temperature for a period ranging from 6 to 8 hours, extracting the above mixture with an ether, washing with brine, drying, on evaporating and column chromatography to obtain 3-tetrahydro-2*H*-2-pyranyloxy-5-(*tert*.butyldimethylsilyloxy)-(1*S*,3*R*,5*R*)-cyclohexan-1-one having formula **8,** 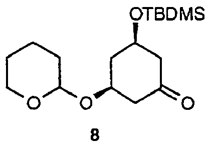
g) reacting a compound of formula **8** with magnesium bromide in an organic solvent at a temperature ranging from 5 to 30 °C for a period ranging from 1 to 12 hours, quenching the above reaction ammonium chloride, separating the organic layer, drying and on evaporating to obtain 3-hydroxy-5-(*tert*-butyldimethyl silyloxy)-(3*S*,5*R*)-cyclohexan-1-one having formula **9,** 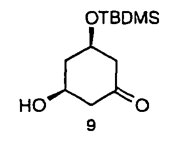
h) reacting a compound of formula 9 with m-chloroperbenzoic acid at room temperature for a period ranging from 16-24 hours, extracting the compound with an orgainc layer, washing with sodium metabisulphite, brine, drying and on evaporation to obtain 6-hydroxymethyl-4-(*tert*-butyldimethylsilyloxy)-(4*R*,6*S*)-tetrahydro-2*H*-2-pyranone having formula **1**.

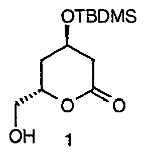

In an embodiment of the present invention the organic solvent used in steps **a, c, e** and **h** for the extraction of the product is selected from the group consisting of ethyl acetate, chloroform, dichloromethane and t-butanol.

In an another embodiment of the present invention the organic solvent used in steps **b, e, f** and **g** for the reaction is selected from the group consisting of ethyl acetate, chloroform, dichloromethane, methanol and diethyl ether.

In yet another embodiment of the present invention the organic base used in step **b** for the reaction is selected from triethylamine and pyridine.

In yet another embodiment of the present invention the buffer used in steps **a** and **c** for the reaction is selected from phosphate buffer and citrate buffer.

In still another embodiment of the present invention the lipase used in steps **a** and **c** for the reaction is selected from the group consisting of pig procain lipase (PPL), pig liver esterase (PLE) and chicken liver acetone powder (CLAP).
In yet another embodiment the present invention provides a compound cis, cis-3-hydroxy-5-methylcarbonyloxy-cyclohexylacetate having formula (**3**),

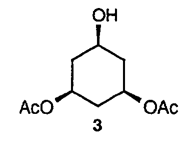

In yet another embodiment the present invention provides a compound *cis, cis*-3-(methylcarbonyloxy)-5-*tert*.butyldimethylsilyloxy) cyclohexylacetate having formula **4,**

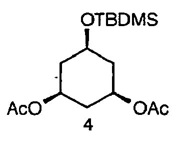

In yet another embodiment the present invention provides a compound 3-hydroxy-5-(*tert*.butyldimethylsilyloxy)-(1S,3R,5R)-cyclohexylacetate having formula **5,**

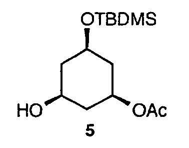

In yet another embodiment the present invention provides a compound 3-tetrahydro-2*H*-2-pyranyloxy-5-(*tert*.butyldimethylsilyloxy)-( 1S,3R,5R)-cyclohexylacetate having formula **6,**

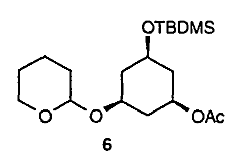

In yet another embodiment the present invention provides a compound 3-tetrahydro-2*H*-2-pyranyloxy-5-(*tert*.butyl dimethylsilyloxy)-(1*S*,3*R*,5*R*)cyclohexan- 1-ol having formula 7,

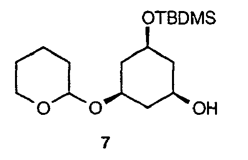

In yet another embodiment the present invention provides a compound 3-tetrahydro-2*H*-2-pyranyloxy-5-(*tert*.butyldimethylsilyloxy)-(1S,3R,5R)-cyclohexan-1-one having formula **8,**

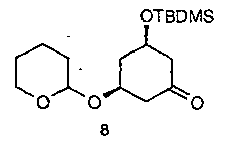

In yet another embodiment the present invention provides a compound 3-hydroxy-5-(*tert*-butyldimethylsilyloxy)-(3S,5R)-cyclohexan-1-one having formula **9,**

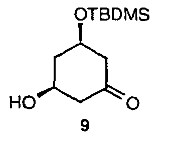

*Cis,cis*-3,5-di(methylcarbonyloxy)cyclohexylacetate (**2**) is prepared by treatment of acetic anhydride with *cis*-1,3,5-cyclohexantriol which is made by known literature procedure (Strong, P. N.; Keana, J. F. W. J Org. Chem. 1975, 40, 956).

The process of the present invention is described hereinbelow with references to the following examples, which are illustrative only and should not be construed to limit the scope of the present invention in any manner.

### Example 1

### Preparation of cis, cis-3-hydroxy-5-methylcarbonyloxy-cyclohexylacetate 3:

Finely powdered *cis, cis*-3,5-di(methylcarbonyloxy)cyclohexylacetate **2** (6.5 parts, 25.19 mmol parts) was suspended in 0.1 M sodium phosphate buffer (pH 7) (135 parts) and stirred vigorously. To the stirred suspension Porcine Liver Esterase (0.110 parts) was added and reaction mixture was stirred vigorously at 30°C for 20 hr. pH of the reaction mixture was monitored at every 2 hrs and was maintained at pH 7 using 1N NaOH solution. After completion of reaction (20 hr), it was extracted with ethyl acetate (2 x 150 parts). Organic layers were combined and washed with brine, dried on anhydrous sodium sulfate and concentrated under vacuum to yield *cis,cis*-3-hydroxy-5-methylcarbonyloxycyclohexylacetate **3** yield (4.8 g parts, 88%).
1H NMR(CDCl₃): δ 1.20-1.58 (qn, 3H), 2.08 (s, 6H), 2.28 (m, 3H), 3.78 (m, 1H), 4.78 (m, 2H)
13C NMR (CDCl₃): δ 21.15, 36.33, 39.93, 64.83, 67.65, 170.42
IR (KBr): 754.60, 884.15, 1029.29, 1140.34, 1250.00, 1367.64, 1738.92, 2871.17, 2953.14, 3445.47
Mass: Base m/e = 96 other m/e: 156, 138, 114, 73, 67, 67, 60, 55
Elemental analysis: calculated for C₁₀H₁₆O₅: C 55.56%, H 7.40% Found : C 55.30%, H 8.00%

### Example 2

### Preparation of cis,cis-3-(methylcarbonyloxy)-5-(tert.butyldimethylsilyloxy) cyclohexylacetate 4

*Cis, cis*-3-hydroxy-5-methylcarbonyloxy-cyclohexylacetate (**3**, 2 parts, 9.26 mmol) and DMAP (0.113 parts, 0.926 mmol) were placed in 100 ml two-necked round bottom flask equipped with dropping funnel and two-way stopcock. It was evacuated and flushed with argon. To it, dry dichloromethane (10 parts) and dry HMPA (2 part) was added and stirred to dissolve. The solution was cooled to -10°C with stirring. To it, solution of *tert*-butyldimethylsilyl chloride in 10 part dry dichloromethane was added dropwise while maintaining temperature below 0°C. Reaction mixture was stirred for 15 min and to it dry triethylamine (2.02 g, 20 mmol) was added dropwise. Reaction mixture was stirred at room temperature for 12 hr. It was then transferred to a separating funnel and washed successively with cold, dil. HCl water, aq. NaHCO₃ and then brine. Organic layer was dried on anhydrous sodium sulfate and solvent was removed under vacuum. Residue was purified by flash column chromatography. (eluent 2-4% ethyl acetate in petroleum ether) to yield *Cis,cis*-3-(methylcarbonyloxy)-5-(*tert*.butyldimethylsilyloxy)cyclohexylacetate (**4**, yield 2.85 parts, 90%).
¹H NMR (CDCl₃): δ 0.06(s, 9H), 0.87 (s, 6H), 1.20-1.45 (m, 3H), 2.03 (s, 6H), 2.2 (m, 3H), 3.38 (m, 1H), 4.73 (m, 2H). 2.3
¹³C NMR (CDCl₃): δ -5.07, 17.61, 25.40, 36.17, 40.40, 65.43, 67.01, 169.64
IR (CHCl₃): 758.90, 838.05, 1034.91, 1106.32, 1246.82, 1368.91, 1734.01, 2858.81, 2955.07
Mass: Base m/e = 117 other m/e: 273, 213, 171, 159, 129, 117, 97, 79, 75, 57
Elemental analysis: calculated for C₂₆H₃₀O₅Si : C 58.185%, H 9.10% Found : C 58.19%, H 9.50%

### Example 3

### Preparation of 3-hydroxy-5-(tert.butyldimethylsilyloxy)-(1S,3R,5R)-cyclohexyl acetate (5)

*Cis, cis*-3-(methylcarbonyloxy)-5-(*tert*.butyldimethylsilyloxy)cyclohexylacetate (**4**, 5 patrs, 147 mmol) was dissolved in *tert*-butanol (20 parts). To the solution, 0.1 M sodium phosphate buffer (230 parts, pH 8) was added and mixture was stirred vigorously. To the stirred emulsion, Porcine liver esterase (0.150 parts) was added and the mixture was stirred vigorously at 30°C for 54 hrs. During reaction pH was maintained at 8 using 1N sodium hydroxide solution. Reaction mixture was extracted with ethyl acetate (3 x 200 parts). Organic layers were combined and washed with brine. It was then dried on anhydrous sodium sulfate and solvent was removed under vacuum. Oily residue contained 3-hydroxy-5-(*tert*.butyldimethylsilyloxy)-(1S,3*R*,5*R*)-cyclohexylacetate **5** along with unreacted **4.** Both were separated by flash column chromatography. *Cis,cis*-3-(methylcarbonyloxy)-5-(*tert*.butyldimethylsilyloxy)cyclohexylacetate recovered: 1.07 parts; 3-hydroxy-5-(*tert*.butyldimethylsilyloxy)-(1*S*,3*R*,5*R*)-cyclohexylacetate (**5**, yield 2.8 parts, 70% based on recovered starting material.
¹H NMR (CDCl₃): δ 0.06 (s, 9H), 0.87 (s, 6H), 1.35-1.60, (m, 3H), 2.06 (s, 3H), 2.15 (m, 3H), 3.7 (m, 2H), 4.75 (m, 1H)
¹³C NMR (CDCl₃): δ -4.60, 18.15, 21.38, 25.90, 39.98, 40.31, 43.93, 65.45, 66.40, 68.17, 170.68
IR (CHCl₃): 758.43, 838.93, 1049.42, 1109.15, 1218.09, 1254.01, 1370.09, 1725.03, 2859.8, 2887.95, 2952.33, 3017.48
Mass: Base m/e = 75 other m/e: 231, 171, 129, 117, 105, 97, 79, 75, 67, 59
Elemental analysis: calculated for C₁₄H₂₈O₄Si : C 58.33%, H 9.72% Found : C 58.15%, H 10.20% Specific rotation [α]_{D} = -4.8 (c 1, CHCl₃) e.e. >95% (determined by chiral HPLC of corresponding Mosher ester. Column: Whelk-O1 [4.0 mm Id x 25 cm] AT-256; λ =254 nm, flow rate: 1 ml/min; mobile phase: Hexane:isopropanol 98:02; retention time for Mosher ester of **5** = 4.59, for Mosher ester of **ent-5** = 4.34).

### Example 4:

### Preparation of 3-tetrahydro-2H-2-pyranyloxy-5-(tert.butyldimethylsilyloxy)-(1S,3R,5R)-cyclohexylacetate ( 6)

3-hydroxy-5-(*tert*.butyldimethylsilyloxy)-(1*S*,3*R*,5*R*)-cyclohexylacetate (**5**, 2.9 parts, 9.73 mmol) was dissolved in dry dichloromethane (30 parts). The solution was cooled below 0°C in ice-salt bath. To the stirred solution, dihydropyran (1 part, 12 mmol) was added and p-toluenesulfonic acid monohydrate (0.1 part) was added as catalyst. Reaction mixture was stirred at -10°C for 2 hr. Reaction was quenched by adding aqueous sodium bicarbonate solution. Both the layers were separated. Aqueous layer was extracted dichloromethane (10 part). Organic layers were combined and washed with water followed by brine wash. It was then dried on anhydrous sodium sulfate and solvent was removed under vacuum. Residue was purified by flash column chromatography to yield 3-tetrahydro-2*H*-2-pyranyloxy-5-(*tert*.butyldimethylsilyloxy)-(1*S*,3*R*,5*R*)-cyclohexylacetate **6** (yield 3.7 parts, 99.5%).
¹H NMR (CDCl₃): δ 0.07 (s, 6H), 0.87 (s, 9H), 1.25-1.90 (m, 9H), 2.04 (s, 3H), 2.05-40 (m, 3H), 3.40-3.75 (m, 3H), 3.86 (m, 1H), 4.55-4.80 (m, 2H)
¹³C NMR (CDCl₃): δ -4.90, 18.00, 19.40, 19.50, 25.20, 25.80, 30.90, 36.80, 40.70, 41.00, 42.80, 62.00, 62.50, 66.00, 68.00, 69.70, 95.00, 96.80
IR (CHCl₃): 752.08, 768.22, 838.35, 1029.76, 1114.56, 1215.63, 1251.94, 1727.19, 2858.86, 2950.80, 3016.74
Mass: Base m/e = 85 other m/e: 231, 211, 171, 159, 129, 117, 105, 101, 85, 79, 75, 67, 55
Elemental analysis: calculated for C₁₉H₃₆O₅Si : C 61.29%, H 9.68% Found : C 61.37%, H 10.03%
Specific rotation [α]_{D} = +1.39 (c 1, CHCl₃)

### Example 5

### Preparation of 3-tetrahydro-2H-2-pyranyloxy-5-(tert.butyldimethylsilyloxy)-(1S,3R,5R)-cyclohexan-1-ol (7)

3-tetrahydro-2*H*-2-pyranyloxy-5-(*tert*.butyldimethylsilyloxy)-( 1*S*,3*R*,5*R*)-cyclohexylacetate (**6**, 3.5 parts, 9.16 mmol) was dissolved in dry methanol (25 parts). To the solution anhydrous potassium carbonate (0.828 parts, 6 mmol) was added and the mixture was stirred at room temperature for 2 hr. Methanol was removed under vacuum and residue was extracted several times with dichloromethane. Dichloromethane layers were combined and washed with water followed by brine wash. It was dried on anhydrous sodium sulfate. Solvent was removed under vacuum and residue was purified by flash column chromatography to yield 3-tetrahydro-2*H*-2-pyranyloxy-5-(*tert*.butyldimethylsilyloxy)-(1*S*,3*R*,5*R*)-cyclohexan-1-ol (**7**, yield 3 parts, 96%).
¹H NMR (CDCl₃): δ 0.06 (s, 6H), 0.87 (s, 9H), 1.35-1.90 (m, 10H), 2.05-2.35 (m, 3H), 3.40-3.75 (m, 4H), 3.80-3.98 (m, 1H), 4.73 (s, 1H)
¹³C NMR (CDCl₃): δ -4.90, 18.00, 19.50, 25.20, 25.80, 30.90, 40.00, 40.70, 42.00, 42.30, 44.70, 62.00, 62.50, 65.80, 66.90, 69.50, 70.00, 96.40, 96.80
IR (CHCl₃): 753.06, 766.83, 838.54, 867.39, 1020.84, 1048.02, 1114.23, 1215.02, 1253.72, 2858.73, 28884.47, 2947.49, 3013.80, 3418.48
Mass: Base m/e = 75 other m/e: 309, 189, 171, 129, 119, 101, 85, 79, 75, 67, 55
Elemental analysis: calculated for C₁₇H₃₄O₄Si : C 61.82%, H 10.30% Found : C 61.83%, H 11.00%
Specific rotation [α]_{D} = +0.93 (c 1, CHCl₃)

### Example 6

### Preparation of 3-tetrahydro-2H-2-pyranyloxy-5-(tert.butyldimethylsilyloxy)-(3R,5R)-cyclohexan-1-one (8)

3-tetrahydro-2*H*-2-pyranyloxy-5-(*tert*.butyldimethylsilyloxy)-( 1*S*,3*R*,5*R*)cyclohexan-1-ol (**7**, 2.85 parts, 8.38 mmol) was dissolved in dry dichloromethane (25 parts) under argon atmosphere. To it, anhydrous sodium acetate (0.2 part, 2.6 mmol) and pyridinium chlorochromate (13 part, 2.6 mmol) were added in one portion and the mixture was stirred under argon atmosphere for 8 hr. Then reaction mixture was diluted with diethyl ether (30 parts) and stirred well. The solution was decanted and the remaining black tar was extracted with diethyl ether (13 x 15 parts). Organic layers were combined and were filtered through small ceilite bed. Then, the organic layer was washed with water (3 x 20 parts) followed by brine wash. Then it was dried on anhydrous sodium sulfate and solvent was removed under vacuum. Residue was purified by flash column chromatography. Yield of 3-tetrahydro-2*H*-2-pyranyloxy-5-(*tert*.butyldimethylsilyl oxy)-(3*R*,5*R*)-cyclohexan-1-one (**8**, 2.24 parts, 80%).
¹H NMR (CDCl₃): δ 0.07 (s, 6H), 0.87 (s, 9H), 1.35-1.95 (m, 8H), 2.2-2.8 (m, 4H), 3.50 (m, 1H), 3.85 (m, 3H), 4.60 & 4.75 (2s, 1H)
¹³C NMR (CDCl₃): δ -4.9, 18.0, 19.5, 19.9, 25.8, 26.0, 30.9, 41.0, 42.5, 47.0, 48.5, 51.5, 62.5, 63.0, 69.0, 69.8, 97.5, 206.5, 207.0
IR (CHCl₃): 858.87, 980.49, 1028.79, 1053.00, 1254.14, 1360.70, 1377.86, 1462.85, 1717.24, 2857.60, 2892.24
Mass: Base m/e = 187, 85 other m/e: 271, 227, 169, 159, 143, 127, 95, 75, 67
Elemental analysis: calculated for C₁₇H₃₂O₄Si : C 62.19%, H 9.75% Found : C 61.9%, H 10.10%
Specific rotation [α]_{D} = +3.71 (c 1, CHCl₃)

### Example 7

### Preparation of 3-hydroxy-5-(tert-butyldimethylsilyloxy)-(3S,5R)-cyclohexan-1-one (9)

3-tetrahydro-2*H*-2-pyranyloxy-5-(*tert*.butyldimethylsilyloxy)-(3*R*,5*R*)-cyclohexan-1-one (**8**, 1 part, 2.959 mmol) was placed in 50 parts two necked round bottom flask equipped with two-way stopcock and rubber septum. Flask was evacuated and flushed with argon. To it, dry ether (10 parts) was added and the resulting solution was stirred vigorously. To the stirred solution magnesium bromide etherate (2.3 parts, 8.9 mmol) was added and the mixture was stirred for 10 hr. The reaction mixture was cooled in ice-bath and reaction was quenched by adding saturated ammonium chloride solution. Both the layers were separated. Aqueous layer was extracted with ether (2 x 10 parts). Organic layers were combined and washed with brine. Then it was dried on anhydrous sodium sulfate and solvent was removed under vacuum. Residue was purified to yield 3-hydroxy-5-(*tert*-butyldimethylsilyloxy)-(3*S*,5*R*)-cyclohexan-1-one (**9**, 0.67 parts, 89%).
¹H NMR (CDCl₃): δ 0.10 (s, 6H), 0.88 (s, 9H), 1.95-2.30 (m, 2H), 2.45-2.78 (m, 4H), 3.95 (d, 1H), 4.36 (m, 1H), 4.56 (m, 1H)
¹³C NMR (CDCl₃): δ -5.28, -5.50, 17.55, 25.34, 38.24, 49.56, 49.89, 68.86, 70.48, 206.78
IR (CHCl₃): 777.13, 835.81, 1010.66, 1045.71, 1095.81, 1254.55, 1381.17, 1413.71, 1464.09, 1715.72.2857.70, 2892.64, 2932.26, 2951.84, 3439.28
Mass: Base m/e = 75 other m/e: 187, 169, 145, 129, 101, 95, 75, 69, 59
Elemental analysis: calculated for C₁₂H₂₄O₃Si : C 59.01%, H 9.83% Found : C 58.65%, H 10.18%
Specific rotation [α]_{D} = +16.20 (c 1, CHCl₃)

### Example 8

### Preparation of 6-hydroxymethyl-4-(tert-butyldimethylsilyloxy)-(4R,6S)-tetrahydro -2H-2-pyranone (1):

3-Hydroxy-5-(*tert*-butyldimethylsilyloxy)-(3*S*,5*R*)-cyclohexan-1-one (**9**, 0.1 part, 0.394 mmol) and 50% 3-chloroperbenzoic acid (0.275 parts, 0.79 mmol) were mixed and kept in dark for 24 hr. Reaction mixture was dissolved in ethyl acetate and washed successively with sodium metabisulfite solution, sodium bicarbonate solution followed by brine wash. It was then dried on anhydrous sodium sulfate and solvent was removed under vacuum. Residue was purified by flash column chromatography to yield white, crystalline 6-hydroxymethyl-4-(*tert*-butyldimethylsilyloxy)-(4*R*,6*S*)-tetrahydro-2*H*-2-pyranone (**1**, yield 0.035 part, 50%).
1H NMR (CDCl₃): δ 0.09, 0.07 (2s, 6H), 0.87 (s, 9H), 1.63 (bs, 1H), 1.74-1.9 (m, 2H), 2.59 (d, 2H, J = 3), 3.65 (dd, 1H, J = 3, 12), 3.88 (dd, 1H, J = 3, 12), 4.36 (m, 1H), 4.79 (m, 1H)
13C NMR (CDCl₃): δ -4.93, 17.93, 25.68, 32.15, 39.26, 63.61, 64.59, 77.07, 170.1
IR (CHCl₃): 666.31, 898.12, 1021.88, 1061.06, 1086.17, 1118.98, 1390.84, 1463.17, 1729.43, 2857.28, 3418.25
Mass: Base m/e = 75 other m/e: 260, 229, 203, 185, 161, 143, 129, 111, 101, 69, 59
Elemental analysis: calculated for C₁₂H₂₄O₄Si : C 55.38%, H 9.20% Found : C 55.42%, H 9.03%
Specific rotation [α]_{D} = +3.0 (c 1, CHCl₃) before crystallization e.e. 86%
[α]_{D} = +1.9 (c 1, CHCl₃) after recrstallization e.e 98% (determined by chiral HPLC of corresponding benzoate derivative, column-Whelk-O1 [4.0 mm Id x 25 cm] AT-256; λ =254 nm, flow rate: 1 ml/min; mobile phase: Hexane:isopropanol 92:08; retention time for benzoate of **1** = 17.48, for benzoate of **ent-1** = 20.10).

## Claims

1. A process for the preparation of 6-hydroxymethyl-4-(*tert*-butyldimethylsilyloxy)-(4R,6S)-tetrahydro-2*H*-2-pyranone of formula **1** wherein the said process comprising :
(a) reacting a compound of formula **2** with a lipase enzyme in a buffer having 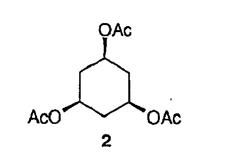 pH ranging between 5 to 7, at a temperature ranging from 25 to 30 °C for a period ranging between 19 to 30 hrs. extracting the reaction mixture with an organic solvent, removing the solvent by evaporation to obtain *cis,cis*-3-hydroxy-5-methylcarbonyloxy-cyclohexylacetate having formula (**3**), 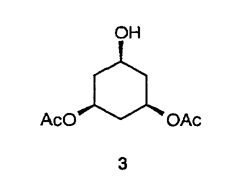
(b) reacting a compound of formula 3 with *tert*-butyldimethylsilylchloride in an organic solvent in the presence of an organic base at a temperature ranging from - 15 to 20°C for a period ranging from 6 to 12 hrs, separating the organic solvent, drying and on evaporation to obtain cis,cis-3-(methylcarbonyloxy)-5-(*tert*.butyldimethylsilyloxy)cyclohexylacetate having formula **4**, 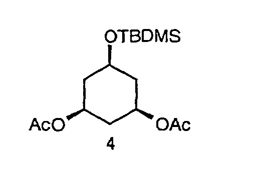
(c) reacting a compound 4 with a lipase enzyme in a buffer having pH in the range of 5 to 8, at a temperature ranging from 25 to 30 °C for a period ranging between 24 to 60 hrs., extracting the mixture with an organic solvent, removing the solvent by evaporation and on column chromatography to obtain 3-hydroxy-5-(*tert*.butyldimethylsilyloxy)-(1*S*,3*R*,5*R*)-cyclohexylace- tate having formula **5**, 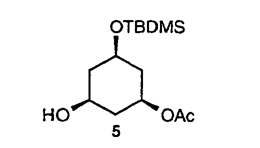
(d) reacting a compound of formula **5** with dihydropyran in an organic solvent in the presence of p-toluene sulphonic acid at a temperature ranging from 5 to -10 °C for a period ranging from 2 to 5 hrs, quenching the above reaction with an aqueous sodium bicarbonate, separating the organic layer, drying, on evaporating and column chromatography to obtain 3-tetrahydro-2*H*-2-pyranyloxy-5-(*tert*.butyldimethylsilyloxy)-(1*S*,3*R*,5*R*)-cyclohexylacetate having formula **6**, 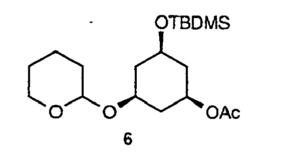
(e) reacting a compound of formula **6** with an anhydrous potassium carbonate in methanol at room temperature for a period ranging from 2 to 6 hrs, evaporating the solvent, extracting with an organic solvent, washing with brine solution, drying, evaporating and column chromatography to obtain 3-tetrahydro-2*H*-2-pyranyloxy-5-(*tert*.butyldimethylsilyloxy)-(1*S*,3*R*,5*R*)-cyclohexan-1-ol having formula **7**, 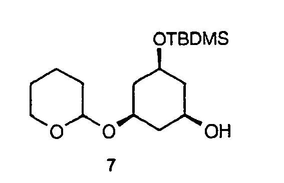
(f) reacting a compound of formula **7** with pyridinium chlorochromate in an organic solvent at room temperature for a period ranging from 6 to 8 hours, extracting the above mixture with an ether, washing with brine, drying, on evaporating and column chromatography to obtain 3-tetrahydro-2*H*-2-pyranyloxy-5-(*tert*.butyldimethylsilyloxy)-(1*S*,3*R*,5*R*)-cyclohexan-1-one having formula **8,** 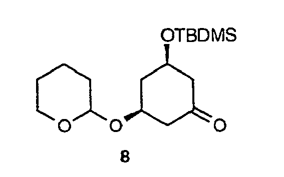
(g) reacting a compound of formula 8 with magnesium bromide in an organic solvent at a temperature ranging from 5 to 30 °C for a period ranging from 1 to 12 hours, quenching the above reaction ammonium chloride, separating the organic layer, drying and on evaporating to obtain 3-hydroxy-5-(*tert*-butyldimethyl silyloxy)-(3*S*,5*R*)-cyclohexan-1-one having formula **9,** 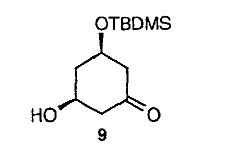
(h) reacting a compound of formula 9 with m-chloroperbenzoic acid at room temperature for a period ranging from 16-24 hours, extracting the compound with an orgainc layer, washing with sodium metabisulphite, brine, drying and on evaporation to obtain 6-hydroxymethyl-4-(*tert*-butyldimethylsilyloxy)-(4*R*,6*S*)-tetrahydro-2*H*-2-pyranone having formula 1.

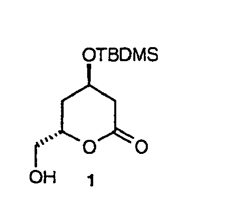

2. A process as claimed in claim 1, wherein the organic solvent used in steps **a, c, e** and **h** for the extraction of the product is selected from the group consisting of ethyl acetate, chloroform, dichloromethane and t-butanol.

3. A process as claimed in claim 1, wherein the organic solvent used in steps **b, e, f** and **g** for the reaction is selected from the group consisting of ethyl acetate, chloroform, dichloromethane, methanol and diethyl ether.

4. A process as claimed in claim 1, wherein the organic base used in step **b** for the reaction is selected from triethylamine and pyridine.

5. A process as claimed in claim 1, wherein the buffer used in steps **a** and **c** for the reaction is selected from phosphate buffer and citrate buffer.

6. A process as claimed in claim 1, wherein the lipase used in steps **a** and **c** for the reaction is selected from the group consisting of pig procain lipase (PPL), pig liver esterase (PLE) and chicken liver acetone powder (CLAP).

7. A compound cis, cis-3-hydroxy-5-methylcarbonyloxy-cyclohexylacetate having formula (**3**).

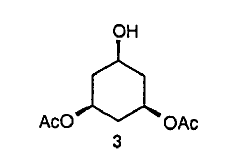

8. A compound *cis, cis*-3-(methylcarbonyloxy)-5-*tert*.butyldimethylsilyloxy) cyclohexylacetate having formula **4**.

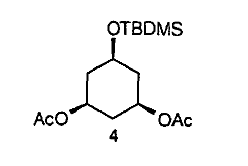

9. A compound 3-hydroxy-5-(*tert*.butyldimethylsilyloxy)-(1S,3R,5R)-cyclohexylacetate having formula **5**

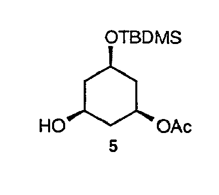

10. A compound 3-tetrahydro-2*H*-2-pyranyloxy-5-(*tert*.butyldimethylsilyloxy)-(1S,3R,5R)-cyclohexylacetate having formula **6**.

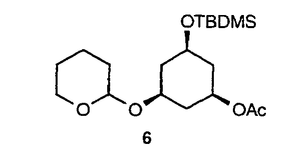

11. A compound 3-tetrahydro-2*H*-2-pyranyloxy-5-(*tert*.butyl dimethylsilyloxy)-(1*S*,3*R*,5*R*)cyaohexan-1-ol having formula 7

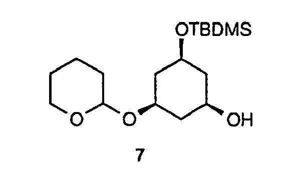

12. A compound 3-tetrahydro-2*H*-2-pyranyloxy-5-(*tert*.butyldimethylsilyloxy)-(1S,3R,5R)-cyclohexan-1-one having formula **8**.

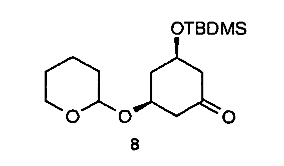

13. A compound 3-hydroxy-5-(*tert*-butyldimethylsilyloxy)-(3S,5R)-cyclohexan-1-one having formula **9.**

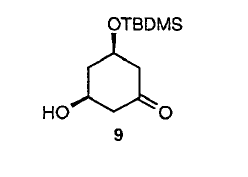

## Patentansprüche

1. Verfahren zur Herstellung von 6-Hydroxymethyl-4-(tert-butyldimethylsilyloxy)-(4R,6S)-tetrahydro-2H-2-pyranon der Formel 1, wobei das Verfahren umfasst:
(a) Umsetzen einer Verbindung der Formel 2 mit einem Lipaseenzym in einem Puffer 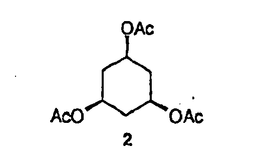 mit einem pH-Wert im Bereich zwischen 5 und 7 bei einer Temperatur im Bereich von 25 bis 30 °C über einen Zeitraum im Bereich zwischen 19 und 30 h, Extrahieren des Reaktionsgemischs mit einem organischen Lösemittel, Entfernen des Lösemittels durch Eindampfen, wobei cis,cis-3-Hydroxy-5-methylcarbonyloxy-cyclohexylacetat der Formel (3) erhalten wird, 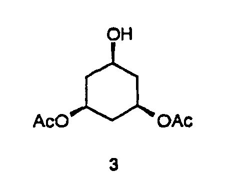
(b) Umsetzen einer Verbindung der Formel 3 mit tert-Butyldimethylsilylchlorid in einem organischen Lösemittel in Gegenwart einer organischen Base bei einer Temperatur im Bereich von -15 bis 20 °C über einen Zeitraum im Bereich von 6 bis 12 h, Abtrennen des organischen Lösemittels, Trocknen und Eindampfen, wobei cis,cis-3-(Methylcarbonyloxy)-5-(tert-butyldimethylsilyloxy)cyclohexylacetat der Formel 4 erhalten wird, 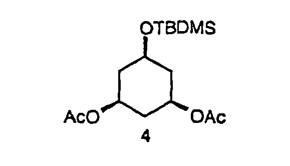
(c) Umsetzen einer Verbindung 4 mit einem Lipaseenzym in einem Puffer mit einem pH-Wert im Bereich von 5 bis 8 bei einer Temperatur im Bereich von 25 bis 30 °C über einen Zeitraum im Bereich zwischen 24 und 60 h, Extrahieren des Gemischs mit einem organischen Lösemittel, Entfernen des Lösemittels durch Eindampfen und durch Säulenchromatographie, wobei 3-Hydroxy-5-(tert-butyldimethylsilyloxy)-(1S,3R,5R)-cyclohexylacetat der Formel 5 erhalten wird, 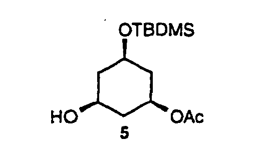
(d) Umsetzen einer Verbindung der Formel 5 mit Dihydropyran in einem organischen Lösemittel in Gegenwart von p-Toluolsulfonsäure bei einer Temperatur im Bereich von 5 bis -10 °C über einen Zeitraum im Bereich von 2 bis 5 h, Quenchen des obigen Reaktionsgemischs mit wässrigem Natriumbicarbonat, Abtrennen der organischen Schicht, Trocknen, wobei bei Eindampfen und Säulenchromatographie 3-Tetrahydro-2H-2-pyranyloxy-5-(tert-butyldimethylsilyloxy)-(1S,3R,5R)-cyclohexylacetat der Formel 6 erhalten wird, 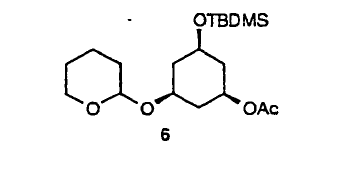
(e) Umsetzen einer Verbindung der Formel 6 mit wasserfreiem Kaliumcarbonat in Methanol bei Raumtemperatur über einen Zeitraum im Bereich von 2 bis 6 h, Abdampfen des Lösemittels, Extrahieren mit einem organischen Lösemittel, Waschen mit Kochsalzlösung, Trocknen, Eindampfen und Durchführen von Säulenchromatographie, wobei 3-Tetrahydro-2H-2-pyranyloxy-5-(tert-butyldimethylsilyloxy)-(1S,3R,5R)-cyclohexan-1-ol der Formel 7 erhalten wird, 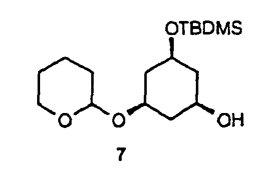
(f) Umsetzen einer Verbindung der Formel 7 mit Pyridiniumchlorchromat in einem organischen Lösemittel bei Raumtemperatur über einen Zeitraum im Bereich von 6 bis 8 h, Extrahieren des obigen Gemischs mit einem Ether, Waschen mit Kochsalzlösung, Trocknen, Eindampfen und Durchführen von Säulenchromatographie, wobei 3-Tetrahydro-2H-2-pyranyloxy-5-(tert-butyldimethylsilyloxy)-(1S,3R,5R)-cyclohexan-1-on der Formel 8 erhalten wird, 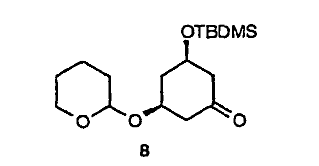
(g) Umsetzen einer Verbindung der Formel 8 mit Magnesiumbromid in einem organischen Lösemittel bei einer Temperatur im Bereich von 5 bis 30 °C über einen Zeitraum im Bereich von 1 bis 12 h, Quenchen des obigen Reaktionsgemischs durch Ammoniumchlorid, Abtrennen der organischen Schicht, Trocknen und Eindampfen, wobei 3-Hydroxy-5-(tert-butyldimethylsilyl-oxy)-(3S,5R)-cyclohexan-1-on der Formel 9 erhalten wird, 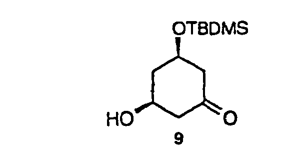
(h) Umsetzen einer Verbindung der Formel 9 mit m-Chlorperbenzoesäure bei Raumtemperatur über einen Zeitraum im Bereich von 16 - 24 h, Extrahieren der Verbindung mit einer organischen Schicht, Waschen mit Natriummetabisulfit, Kochsalzlösung, Trocknen und Eindampfen, wobei 6-Hydroxymethyl-4-(tert-butyldimethyl-silyloxy)-(4R,6S)-tetrahydro-2H-2-pyranon der Formel 1 erhalten wird.

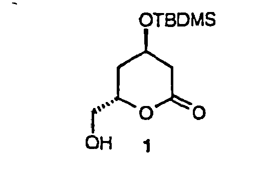

2. Verfahren gemäß Anspruch 1, wobei das in den Stufen a, c, e und h für die Extraktion des Produkts verwendete organische Lösemittel aus der Gruppe von Ethylacetat, Chloroform, Dichlormethan und tert-Butanol ausgewählt ist.

3. Verfahren gemäß Anspruch 1, wobei das in den Stufen b, e, f und g für die Reaktion verwendete organische Lösemittel aus der Gruppe von Ethylacetat, Chloroform, Dichlormethan, Methanol und Diethylether ausgewählt ist.

4. Verfahren gemäß Anspruch 1, wobei die in Stufe b für die Reaktion verwendete organische Base aus Triethylamin und Pyridin ausgewählt ist.

5. Verfahren gemäß Anspruch 1, wobei der in den Stufen a und c für die Reaktion verwendete Puffer aus einem Phosphatpuffer und einem Citratpuffer ausgewählt ist.

6. Verfahren gemäß Anspruch 1, wobei die in den Stufen a und c für die Reaktion verwendete Lipase aus der Gruppe von Schweine-Procainlipase (Pig Procain Lipase, PPL), Schweineleberesterase (Pig Liver Esterase, PLE) und Hähnchenleber-Acetonpulver (Chicken Liver Acetone Powder, CLAP) ausgewählt ist.

7. Die Verbindung cis,cis-3-Hydroxy-5-methylcarbonyloxy-cyclohexylacetat der Formel (3).

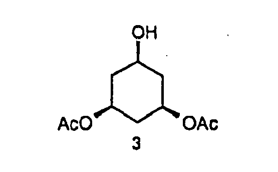

8. Die Verbindung cis,cis-3-(Methylcarbonyloxy)-5-tert-butyldimethylsilyloxy)cyclohexylacetat der Formel 4.

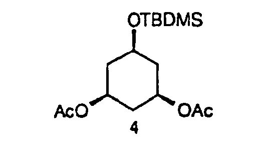

9. Die Verbindung 3-Hydroxy-5-(tert-butyldimethylsilyloxy)-(1S,3R,5R)-cyclohexylacetat der Formel 5.

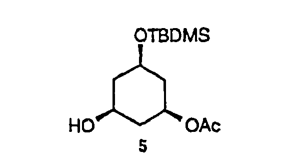

10. Die Verbindung 3-Tetrahydro-2H-2-pyranyloxy-5-(tert-butyldimethylsilyloxy)-(1S,3R,5R))-cyclohexylacetat der Formel 6.

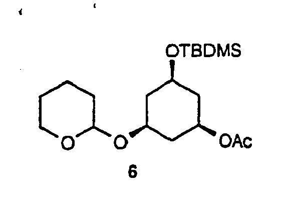

11. Die Verbindung 3-Tetrahydro-2H-2-pyranyloxy-5-(tert-butyldimethylsilyloxy)-(1S,3R,5R)-cyclohexan-1-ol der Formel 7.

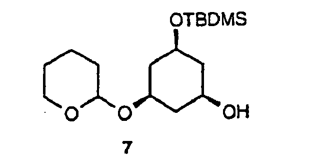

12. Die Verbindung 3-Tetrahydro-2H-2-pyranyloxy-5-(tert-butyldimethylsilyloxy)-(1S,3R,5R)-cyclohexan-1-on der Formel 8.

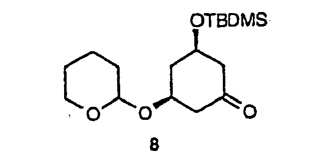

13. Die Verbindung 3-Hydroxy-5-(tert-butyldimethylsilyloxy)-(3S,5R)-cyclohexan-1-on der Formel 9.

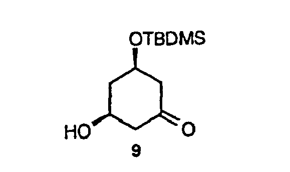

## Revendications

1. Procédé pour la préparation de la 6-hydroxyméthyl-4-(tert-butyldiméthylsilyloxy)-(4R,6S)-tétrahydro-2H-2-pyranone de formule 1, dans lequel ledit procédé comprend :
(a) la réaction d'un composé de formule 2 avec une 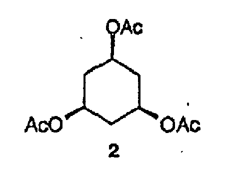 enzyme lipase dans un tampon ayant un pH allant de 5 à 7, à une température allant de 25 à 30 °C pendant une durée allant de 19 à 30 heures, l'extraction du mélange réactionnel avec un solvant organique, l'élimination du solvant par évaporation afin d'obtenir l'acétate de cis,cis-3-hydroxy-5-méthylcarbonyloxy-cyclohéxyle ayant la formule (3), 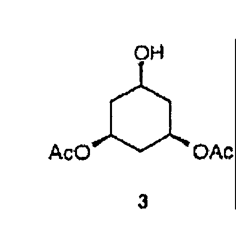
(b) la réaction d'un composé de formule 3 avec du chlorure de tert-butyldiméthylsilyle dans un solvant organique en présence d'une base organique à une température allant de -15 à 20 °C pendant une durée allant de 6 à 12 heures, la séparation du solvant organique, le séchage et l'évaporation pour obtenir l'acétate de cis,cis-3-(méthylcarbonyloxy)-5-(tert.butyldiméthylsilyloxy)cyclohexyle ayant la formule 4, 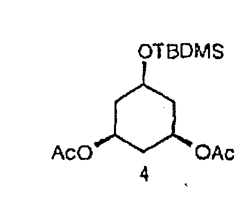
(c) la réaction d'un composé 4 avec une enzyme lipase dans un tampon ayant un pH dans la gamme allant de 5 à 8, à une température allant de 25 à 30 °C pendant une durée allant de 24 à 60 heures, l'extraction du mélange avec un solvant organique, l'élimination du solvant par évaporation et la chromatographie sur colonne pour obtenir l'acétate de 3-hydroxy-5-(tert.butyldiméthylsilyloxy)-(1S,3R,5R)-cyclohexyle ayant la formule 5, 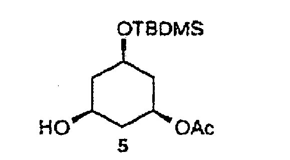
(d) la réaction d'un composé de formule 5 avec du dihydropyrane dans un solvant organique en présence d'acide p-toluène sulfonique à une température allant de 5 à -10 °C pendant une durée allant de 2 à 5 heures, l'arrêt (« quenching ») de la réaction précédente par du bicarbonate de sodium aqueux, la séparation de la couche organique, le séchage, l'évaporation et la chromatographie sur colonne afin d'obtenir l'acétate de 3-tétrahydro-2H-2-pyranyloxy-5-(tert.butyldiméthylsilyloxy)-(1S,3R,5R)-cyclohexyle ayant la formule 6, 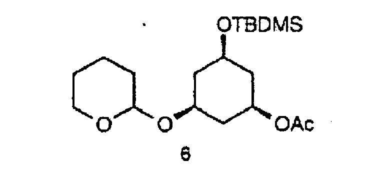
(e) la réaction d'un composé de formule 6 avec du carbonate de potassium anhydre dans du méthanol à température ambiante pendant une durée allant de 2 à 6 heures, l'évaporation du solvant, l'extraction avec un solvant organique, le lavage avec une solution de saumure, le séchage, l'évaporation et la chromatographie sur colonne pour obtenir le 3-tétrahydro-2H-2-pyranyloxy-5-(tert.butyldiméthylsilyloxy)-(1S,3R,5R)-cyclohexanol-1 ayant la formule 7, 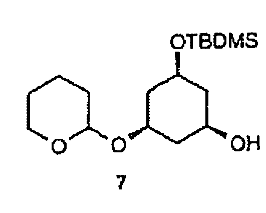
(f) la réaction d'un composé de formule 7 avec du chlorochromate de pyridinium dans un solvant organique à température ambiante pendant une durée allant de 6 à 8 heures, l'extraction du mélange précédent avec un éther, le lavage avec de la saumure, le séchage, l'évaporation et la chromatographie sur colonne pour obtenir la 3-tétrahydro-2H-2-pyranyloxy-5-(tert.butyldiméthylsilyloxy)-(1S,3R,5R)-cyclohexanone-1 ayant la formule 8, 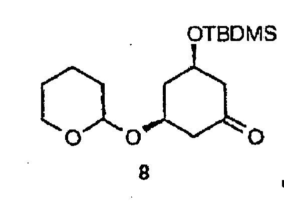
(g) la réaction d'un composé de formule 8 avec du bromure de magnésium dans un solvant organique à une température allant de 5 à 30 °C pendant une durée allant de 1 à 12 heures, l'arrêt (« quenching ») de la réaction précédente avec du chlorure d'ammonium, la séparation de la couche organique, le séchage et l'évaporation pour obtenir la 3-hydroxy-5-(tert-butyldiméthylsilyloxy)-(3S,5R)-cyclohexanone-1 ayant la formule 9, 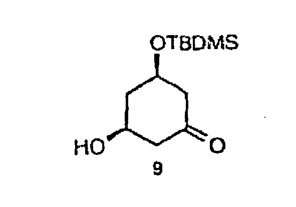
(h) la réaction d'un composé de formule 9 avec de l'acide m-chloroperbenzoïque à température ambiante pendant une durée allant de 16 à 24 heures, l'extraction du composé à l'aide d'une couche organique, le lavage avec du métabisulfite de sodium, de la saumure, le séchage et l'évaporation pour obtenir la 6-hydroxyméthyl-4-(tert-butyldiméthylsilyloxy)-(4R,6S)-tétrahydro-2H-2-pyranone ayant la formule 1.

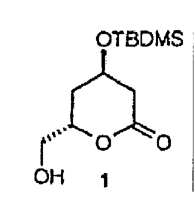

2. Procédé selon la revendication 1, dans lequel le solvant organique utilisé pour l'extraction du produit dans les étapes a, c, e et h est choisi dans le groupe constitué par l'acétate d'éthyle, le chloroforme, le dichlorométhane et le t-butanol.

3. Procédé selon la revendication 1, dans lequel le solvant organique utilisé pour la réaction dans les étapes b, e, f et g est choisi dans le groupe constitué par l'acétate d'éthyle, le chloroforme, le dichlorométhane, le méthanol et l'éther de diéthyle.

4. Procédé selon la revendication 1, dans lequel la base organique utilisée pour la réaction dans l'étape b est choisie parmi la triéthylamine et la pyridine.

5. Procédé selon la revendication 1, dans lequel le tampon utilisé pour la réaction dans les étapes a et c est choisi parmi un tampon phosphate et un tampon citrate.

6. Procédé selon la revendication 1, dans lequel la lipase utilisée pour la réaction dans les étapes a et c est choisie dans le groupe constitué par la lipase de procaïne de porc (PPL), l'esterase de foie de porc (PLE) et la poudre acétonique de foie de poulet (CLAP).

7. Composé acétate de cis,cis-3-hydroxy-5-méthylcarbonyloxy-cyclohexyle ayant la formule (3).

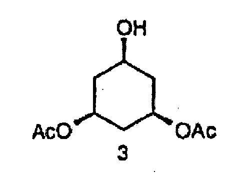

8. Composé acétate de cis,cis-3-(méthylcarbonyloxy)-5-(tert.butyldiméthylsilyloxy)-cyclohexyle ayant la formule 4.

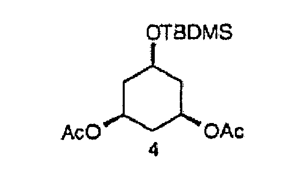

9. Composé acétate de 3-hydroxy-5-(tert.butyldiméthylsilyloxy)-(1S,3R,5R)-cyclohexyle ayant la formule 5.

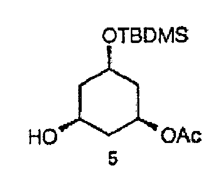

10. Composé acétate de 3-tétrahydro-2H-2-pyranyloxy-5-(tert.butyldiméthylsilyloxy)-(1S,3R,5R)-cyclohexyle ayant la formule 6.

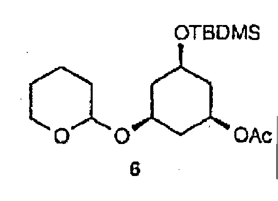

11. Composé 3-tétrahydro-2H-2-pyranyloxy-5-(tert.butyldiméthylsilyloxy)-(1S,3R,5R)cyclohexanol-1 ayant la formule 7.

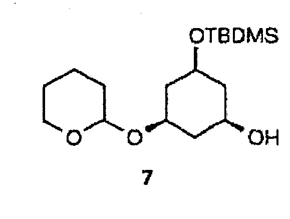

12. Composé 3-tétrahydroxy-2H-2-pyranyloxy-5-(tert.butyldiméthylsilyloxy)-(1S,3R,5R)-cyclohexanone-1 ayant la formule 8.

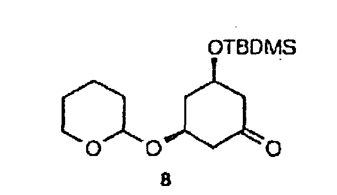

13. Composé 3-hydroxy-5-(tert-butyldiméthylsilyloxy)-(3S,5R)-cyclohexanone-1 ayant la formule 9.
